# EUROPEAN PATENT SPECIFICATION

(11) **EP 2 414 037 B1**
(45) Date of publication and mention of the grant of the patent: **28.10.2015**
(21) Application number: 09842767.7
(22) Date of filing: 31.03.2009
(51) Int. Cl.: A61N 1/362, A61N 1/05, A61N 1/37

(54) **IMPLANTED HEART-STIMULATION DEVICE ENABLING CHARGE BALANCE AFTER STIMULATION SEQUENCE**
LADUNGSGLEICHGEWICHT ERMÖGLICHENDE IMPLANTIERTE HERZSTIMULATIONSVORRICHTUNG NACH EINER STIMULATIONSSEQUENZ
DISPOSITIF DE STIMULATION CARDIAQUE IMPLANTÉ PERMETTANT UN ÉQUILIBRE DE CHARGE APRÈS UNE SÉQUENCE DE STIMULATION

(43) Date of publication of application: 08.02.2012
(73) Proprietor: St. Jude Medical AB, 175 84 Järfälla (SE)
(72) Inventor: EDVINSSON, Jörgen, 192 73 Sollentuna (SE)
(74) Representative: Sjölander, Henrik
(86) International application number: PCT/SE2009/000168
(87) International publication number: WO 2010/114428

(56) References cited:
- EP-A1- 0 308 536
- WO-A1-00/27465
- WO-A1-2004/012813
- US-A- 4 114 627
- US-A- 4 991 583
- US-A- 5 486 201
- US-A- 5 735 883
- US-A1- 2002 147 477
- US-A1- 2004 210 270
- US-A1- 2008 015 641
- US-B2- 7 450 990

## Description

### Technical field

The present invention is generally related to implantable medical devices, in particular to implantable medical devices for heart stimulation, such as pacemakers and similar cardiac rhythm management devices. Specifically, the present invention relates to an implantable medical device with an improved function for maintaining charge balance after the delivery of a stimulation sequence.

### Background

When functioning properly, the human heart maintains its own intrinsic rhythm and is capable of pumping adequate amounts of blood through the circulatory system. However, some individuals suffer from irregular cardiac rhythms, or cardiac arrhythmias, that result in diminished blood circulation. These patients often require cardiac rhythm assistance in the form of a cardiac rhythm management system, such as a pacemaker or a cardioverter or defibrillator, which delivers therapy to their hearts. A pacemaker is in general implanted in the patient, and delivers timed sequences of electrical stimuli, referred to as stimulation pulses or pace pulses, to selected sites in the heart, e.g. selected chambers, generally via a lead wire or catheter (a medical lead) including one or more electrodes disposed in or about the heart, for, e.g., evoking or coordinating heart chamber contractions or modifying the way the heart chambers contract during a cardiac cycle, thereby allowing for treatment of cardiac diseases and dysfunctions. A cardioverter or defibrillator is capable of delivering a high energy electrical stimuli to the heart, e.g. for treating cardiac arrhythmias, such as tachyarrhythmias, or hearts that beat too fast. For example, tachyarrhythmias may cause a diminished blood circulation, because the heart is not allowed sufficient time to fill with blood before contracting to expel the blood, and hence the heart pumps a reduced amount of blood through the circulatory system. The cardioverter or defibrillator interrupts tachyarrhythmias by the high energy electrical stimuli, allowing for a normal heart rhythm to be reestablished.

A heart-stimulation device, such as a pacemaker as mentioned above, generally comprises an anode and a cathode disposed at sites on or about the heart. A stimulation pulse is generated by increasing the potential of the anode to a controlled potential relative to the cathode during a stimulation period, whereby a current starts flowing from the anode to the cathode through the patient. In general, a direct current blocking element, typically a capacitor, is arranged in series with the current path at the cathode, which capacitor is capable of being charged and discharged. During the stimulation period the capacitor may thus be charged. The charge stored in the capacitor may subsequently be recharged through the patient during a discharge period. Ideally, the same amount of charge that passed through the patient during the stimulation period is passed back through the patient during the discharge period. In this way, there is an equal amount of charge flowing from the anode to the cathode during the stimulation period and from the cathode to the anode during the discharge period, thereby maintaining charge neutrality of the stimulation channel at the end of the stimulation sequence.

In a healthy heart, intrinsic signals originate in the sinoatrial node in the upper right atrium and travel through and depolarize the atrial tissue such that contractions of the right and left atria are triggered. The intrinsic atrial signals are received by the atrioventricular node, which in turn triggers a ventricular intrinsic signal that travels through and depolarizes the ventricular tissue such that contractions of the right and left ventricles are triggered substantially simultaneously. For patients having cardiac conduction defects and congestive heart failure, such cardiac depolarizations are generally not conducted in a timely manner. In such cases, the right and left heart chambers do not contract in optimum synchrony with each other, and cardiac function suffers due to these conduction defects. For example, the ventricles may beat slightly out of phase. This asynchrony may greatly reduce the efficiency of the ventricles, in particular in patients already having weak hearts.

Coordination therapy, such as cardiac resynchronization therapy (or biventricular pacing), may require issuing two or more stimulation pulses in different stimulation channels with very short time intervals between the stimulation pulses at multiple electrode sites positioned at more than one site in or on a heart, in order for the regions to contract in a coordinated fashion. For example, coordination therapy may require applying stimulation pulses at sites situated on one or both ventricles or at multiple sites within one or more ventricles for coordinating the ventricular contraction sequence.

Hence, implantable heart stimulators that provide stimulation pulses to selected locations in the heart have been developed for the treatment of cardiac diseases and dysfunctions, for example to influence the manner and degree to which the heart chambers contract during a cardiac cycle in order to promote the efficient pumping of blood.

Conventionally, at least some of the circuits and components, e.g. transistors, of heart-stimulation devices, such as pacemakers, may be implemented as a semiconductor device on a semiconductor substrate, e.g. as an integrated circuit (IC). Semiconductor devices generally exhibit parasitic structures, by which it is meant a portion of a semiconductor device structurally resembling some other semiconductor device and potentially causing the semiconductor device to enter unintended modes of operation when subjected to conditions outside its normal operating range. For instance, whenever an n-doped region and a p-doped region on an IC are situated in close proximity, a parasitic p-n diode may be formed in the p-n junction. If a large enough bias is placed on the junction, a parasitic p-n diode is formed, and current will flow over the parasitic p-n diode. In normal operation of the device, the formation of the parasitic p-n diode may be undesirable.

Consider now a conventional implantable heart-stimulation device including circuits or components implemented on a semiconductor substrate, and the case where cardiac stimulations are required substantially simultaneously at multiple sites in a heart, and where multiple stimulation channels are used, and the stimulation periods are required to be shorter than the respective discharge periods. This scenario is common, for example, in cardiac resynchronization therapy. In this case, each stimulation channel requires a direct current blocking element, such as a coupling capacitor, and the potentials over the coupling capacitors may be high, as the amount of charge flowing through the stimulation channels may be large. When using the device ground as the common discharge node and using multiple coupling capacitors which are not fully discharged one by one, the n-well potential may become less than a parasitic diode threshold voltage below the ground potential, which in turn will lead to current leaking from the substrate to the n-well. The charge lost from one or more coupling capacitors should be discharged back through the respective stimulation channel during the discharge sequence in order to maintain charge neutrality of the respective stimulation channel, and thus, according to the above scenario, charge neutrality of the stimulation device may not be maintained after a stimulation sequence for a stimulation channel has been performed.

Thus, in conventional multi-channel heart-stimulation devices, under certain operating conditions there may be a risk for the heart-stimulation device entering undesired modes of operation. In severe cases this may lead to deterioration of or damage to the electrical components of the heart- stimulation device, which might endanger the health of the patient treated by the heart-stimulation device.

There is thus a need within the art for an improved heart-stimulation device that reduces or eliminates the above-mentioned problems.

EP 0 308 536 discloses a pacemaker having a plurality of solid state switches. During a pacing mode a first reference electrode is switchably connected to the most positive battery potential V_{DD}. During a sensing mode of operation the first reference electrode is switchably connected to -0.5 V. The return electrode can selectively be either the pacemaker case or a ring electrode. During a fast discharge time period the return electrode is disconnected from V_{DD} and connected to the proximal side of a coupling capacitor through which the pacing pulse has passed. Also the most negative battery potential V_{SS} is switchably connected to the proximal side of the coupling capacitor.

US 2004/0210270 actively drives the discharge of blocking capacitors in an apparatus for electrically stimulating tissue, thereby reducing the discharge time. Actively discharging the blocking capacitors may be accomplished with an asymmetric reverse pulse.

US 5,735,883 discloses a pacemaker for detecting capture or adjusting the strength or duration of pacing pulses by assessing the mechanical evoked response that may be distinctly sensed through impedance sensing, pressure sensing or plethysmography.

US 5,486,201 discloses an active discharge circuit for use within a pacemaker, rapidly discharges a coupling capacitor connected between a therapy circuit and body tissue. The active discharge circuit has a switching device, a charge transfer capacitor and a clock. In response to a clock signal the switching device sequentially and repeatedly couples the charge transfer capacitor to a discharge voltage supply and then couples the charge transfer capacitor to the coupling capacitor.

### Summary

In accordance with the above, an object of the present invention is to provide an improved heart-stimulation device capable of multi-site stimulation.

A further object of the present invention is to provide a heart- stimulation device capable of multi-site stimulation that reduces the risk of charge neutrality not being maintained at the end of a stimulation sequence.

These and other objects of the present invention are achieved by a device as defined by the independent claims. Further embodiments are defined by the dependent claims. Additional objects of the present invention will become apparent through the following description.

The methods disclosed herein do not form part of the invention and are merely provided to illustrate the functions and intended use of the device according to the invention.

The present invention is based on the insight that after a stimulation sequence has been terminated, i.e. a sequence of stimulation pulses have been delivered via a plurality of stimulation channels in a heart-stimulation device, where each of the stimulation channels comprises at least one direct current blocking element, such as a capacitor, that may be charged and discharged, the stimulation channels may advantageously be discharged in successive steps, where each step comprises a partial discharge of the respective stimulation channel, according to an appropriate sequence, until the voltage of each stimulation channel is less than a predetermined voltage level. As will be further described in the following, such an arrangement advantageously facilitates maintaining charge balance in each of the stimulation channels after the duration of a stimulation sequence.

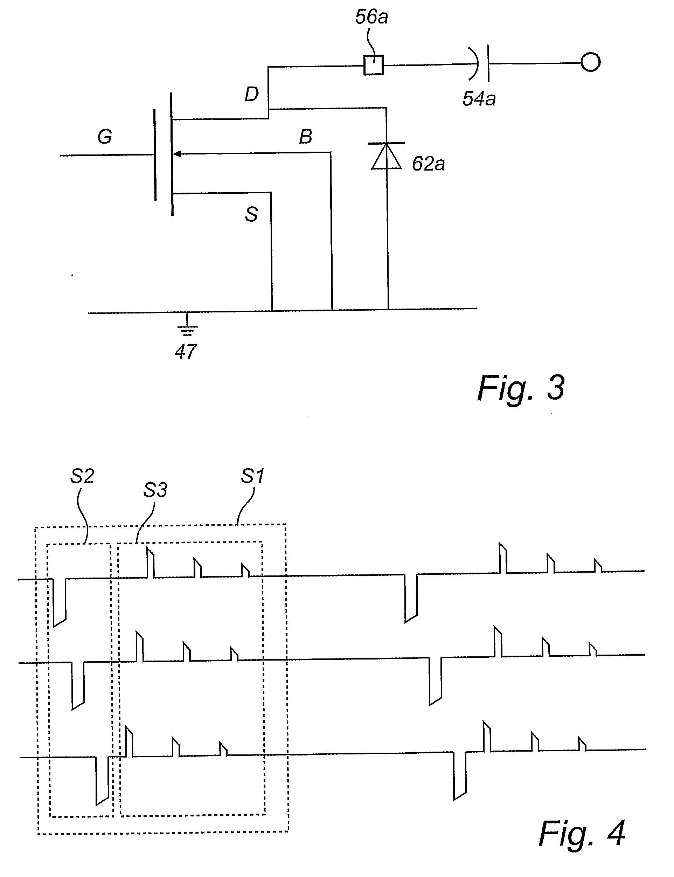

In the context of the present invention, by "stimulation sequence" it is meant a sequence of events comprising stimulation during a stimulation period, including delivery of stimulation pulses via stimulation channels, and discharge during a discharge period, including discharge of the stimulation channels (to be illustrated in the following description with reference to Fig. 4).

According to a first aspect of the present invention, there is provided an implantable heart-stimulation device comprising a pulse generator arranged to generate stimulation pulses, the pulse generator being connectable to at least one medical lead including a plurality of electrodes. The implantable heart-stimulation device further comprises a plurality of stimulation channels for delivering stimulation pulses via the electrodes, wherein each stimulation channel includes at least one coupling capacitor, and a control unit adapted to repeatedly perform a partial discharge of coupling capacitors of the stimulation channels in accordance with a predetermined, temporally non- overlapping sequence. The control unit is further adapted to perform the repeated partial discharge of coupling capacitors of the stimulation channels until each of the coupling capacitors has a voltage less than a predetermined voltage level. Each partial discharge has a predetermined discharge period.

Such a heart-stimulation device enables that charge neutrality in the device at the end of a stimulation sequence with semi-simultaneous stimulations, even when charge transfer is high (e.g., high stimulation amplitudes, long pulse widths and relatively low stimulation impedance), may be maintained. This is achieved by the repeated partial discharge of the coupling capacitors of the stimulation channels, whereby the voltages over the coupling capacitors of the stimulation channels are decreased step-wise in a concerted manner and not decreased to the predetermined voltage level (e.g., zero voltage) one by one, each in a single step. In such a heart-stimulation device, the voltages of a parasitic structure, such as a parasitic diode, formed between a coupling capacitor and the semiconductor substrate becomes significantly less compared to the case where the coupling capacitors are discharged one by one, each in a single step. Thereby, charge loss from the coupling capacitors during the discharge of these elements may be reduced or eliminated. In turn, this allows for alleviating or eliminating the tendency or risk for the heart-stimulation device to enter undesired modes of operation during operation, which, as already discussed above, in severe cases may lead to deterioration of or damage to the electrical components of the heart-stimulation device, possibly endangering the health of the patient being treated by the heart-stimulation device.

There is provided a method for operating an implantable heart-stimulation device comprising a pulse generator arranged to generate stimulation pulses, which pulse generator is connectable to at least one medical lead including a plurality of electrodes, and a plurality of stimulation channels for delivering stimulation pulses via the electrodes, wherein each stimulation channel includes at least one coupling capacitor. The method comprises the step of repeatedly performing a partial discharge of coupling capacitors of stimulation channels according to a predetermined, temporally non-overlapping sequence until each of the coupling capacitors has a voltage less than a predetermined voltage level. According to the present invention, each partial discharge has a predetermined discharge period.

By such a method there is achieved advantages similar or identical to the advantages achieved by the heart-stimulation device according to the present invention as described in the foregoing and in the following.

There is provided an implantable heart-stimulation system comprising a heart-stimulation device according to the first aspect of the present invention or embodiments thereof and at least one medical lead that includes a plurality of electrodes. The at least one medical lead may be adapted to be used in conjunction with the heart-stimulation device.

By such a heart-stimulation system there is achieved a medical system for delivering therapy to patients suffering from cardiac disease using a heart-stimulation device according to the present invention having the advantages as described in the foregoing and in the following.

There is provided a computer program comprising computer code arranged to perform a method as described above. Furthermore, there is provided a computer readable digital storage medium on which there is stored a computer program comprising computer code arranged to perform a method as described above.

By such a computer program and storage medium, respectively, there is provided a means for implementing the method as described above and an easily portable means for implementing the method as described above, respectively, thus achieving advantages similar or identical to the advantages of the method as described above.

There is provided an integrated circuit comprising a pulse generator arranged to generate stimulation pulses, said pulse generator being connectable to at least one medical lead including a plurality of electrodes. The integrated circuit is adapted to be integrated in an implantable heart-stimulation device according to the first aspect of the present invention or embodiments thereof, thus achieving advantages similar or identical to the advantages of the heart-stimulation device according to the first aspect of the present invention or embodiments thereof.

According to an exemplifying embodiment of the present invention, the coupling capacitors of the stimulation channels may be repeatedly partially discharged one stimulation channel at a time while alternating between the stimulation channels.

By such a configuration, there is even further assured that the voltages of the coupling capacitors of the stimulation channels are decreased step-wise in a concerted manner and not decreased to the predetermined voltage level (e.g., zero voltage) one by one, each in a single step. Thereby, charge loss from the coupling capacitors during the discharge of these elements may be reduced or eliminated.

According to another exemplifying embodiment of the present invention, the partial discharges for the stimulation channels may be controlled such that the total discharge period for each stimulation channel corresponds to a predetermined total discharge period.

Thus, the total discharge period for each stimulation channel is allowed to amount to a predetermined total discharge period, which for example may be set to a value such that the coupling capacitor of the respective stimulation channel may reach a substantially completely discharged state during the total discharge period. Consequently, during operation a substantially complete discharge of the at least one coupling capacitor of each stimulation channel may be ensured, regardless of the capacity of the coupling capacitor to store charge (i.e., its capacitance).

According to yet another exemplifying embodiment of the present invention, each coupling capacitor may comprise at least one coupling capacitor. Accordingly, there is achieved a coupling capacitor that is flexible with regards to capacity requirements and relatively inexpensive.

According to yet another exemplifying embodiment of the present invention, each of the plurality of stimulation channels includes an anode and a cathode, and a coupling capacitor is located between the anode and the cathode. The partial discharge of a coupling capacitor of the respective stimulation channel is effectuated by connecting the anode side and the cathode side of the coupling capacitor to a common electrical node during a predetermined discharge period.

In this manner, there is achieved a heart-stimulation device having a plurality of stimulation channels including components that are relatively easy to implement, e.g. on a semiconductor substrate such as an integrated circuit, without the need for custom-built components, as well as adaptable with regards to controlling the delivery of the stimulation pulses and/or the discharge of the stimulation channels.

According to yet another exemplifying embodiment of the present invention, stimulation pulses may be delivered via the stimulation channels according to a predetermined sequence.

In this manner, there is achieved a versatile and adaptable heart-stimulation device with regards to delivery of stimulation pulses via the stimulation channels to selected sites in or about the heart of a patient in order to deliver therapy to the heart, thus also allowing for treating a number of different cardiac disorders.

The circuits and/or components of a heart-stimulation device according to an embodiment of the present invention may be implemented on a semiconductor substrate, such as an integrated circuit.

According to yet another exemplifying embodiment of the present invention, the discharge period may be such that by the partial discharge the voltage of the at least one coupling capacitor of the respective stimulation channel decreases by a voltage less than a threshold voltage of a parasitic structure that may be formed between the semiconductor substrate and the coupling capacitor.

According to yet another exemplifying embodiment of the present invention, the predetermined voltage level may be less than or equal to a threshold voltage of a parasitic structure formed between the semiconductor substrate and the respective at least one coupling capacitor.

By these two configurations described immediately above, when using a plurality of stimulation channels for stimulating the heart of a patient, the risk of charge leaking from a coupling capacitor of a stimulation channel to the semiconductor substrate during or after the discharge of the coupling capacitor is further minimized, and thus the risk of charge neutrality not being maintained at the end of a stimulation sequence is further decreased.

With reference to e.g. the two embodiments described immediately above, such a parasitic structure may for example comprise a parasitic p-n diode.

According to yet another exemplifying embodiment of the present invention, the change in voltage of a coupling capacitor of at least one of the stimulation channels resulting from a partial discharge of said coupling capacitor may be compared, and, on the basis of the comparison thus performed, the discharge period for the at least one stimulation channel may be adjusted.

Thus, the discharge period for each stimulation channel may be adaptively controlled on the basis of the momentaneous voltage of the coupling capacitor of the respective stimulation channel, in response to changing operating conditions, etc. The adjustment of the discharge period for a particular stimulation channel (i.e. the partial discharge period) may be performed at any time, even during a sequence of partial discharges for the particular stimulation channel.

In particular, in accordance with the embodiment of the present invention described immediately above, the magnitude of the adjustment of a partial discharge period associated with a particular stimulation channel may depend on a change in voltage of the coupling capacitor of the particular stimulation channel, which results from a partial discharge of the coupling capacitor associated therewith. Such an adjustment of the partial discharge period for a particular stimulation channel may be performed at any suitable time, for example within a time period during which a sequence of partial discharges of the coupling capacitor associated with the stimulation channel is being carried out. Consequently, the length of the partial discharge period may be adjusted dynamically during operation of the heart-stimulation device, thereby allowing for increased versatility and adaptability with regards to operation of the heart-stimulation device for the purpose of delivering therapy to a patient's heart.

According to yet another exemplifying embodiment of the present invention, the potential of the anode side and the cathode side of a coupling capacitor of at least one stimulation channel may be compared, and, on the basis of the comparison, the discharge period for the at least one stimulation channel may be adjusted.

According to yet another exemplifying embodiment of the present invention, voltages of coupling capacitors of a number of stimulation channels may be compared, and, on the basis of the comparison, the discharge period for one or more of those stimulation channels may be adjusted.

By the two configurations described immediately above, there is provided even further possibilities to control the operation of the heart-stimulation device, particularly to adaptively control the discharge period for one or more stimulation channels on the basis of the differential voltage and/or the voltage of the coupling capacitors of the respective stimulation channels. Thereby, the versatility and flexibility of the heart-stimulation device with regards to operation may be further increased.

According to an exemplifying embodiment of the present invention, the integrated circuit may further comprise at least one coupling capacitor adapted to be included in a stimulation channel arranged to deliver stimulation pulses via the electrodes.

Further objects and advantages of the present invention will be discussed below by means of exemplifying embodiments.

### Brief description of the drawings

Exemplifying embodiments of the invention will be described below with reference to the accompanying drawings, in which:
Fig. 1 is a schematic illustration of an implantable heart-stimulation device according to an exemplifying embodiment of the present invention;
Fig. 2 is schematic circuit diagram of an implantable heart-stimulation device according to another exemplifying embodiment of the present invention;
Fig. 3 is a section of the circuit diagram in Fig. 2;
Fig. 4 are schematic voltage diagrams showing exemplifying stimulation sequences according to an exemplifying embodiment of the present invention;
Fig. 5a are schematic timing diagrams showing exemplifying stimulation sequences according to an exemplifying embodiment of the present invention;
Fig. 5b are schematic voltage diagrams according to an exemplifying embodiment of the present invention;
Figs. 6a-6f are schematic voltage diagrams according to exemplifying embodiments of the present invention; and
Figs. 7a-7f are schematic voltage diagrams according to exemplifying embodiments of the present invention.

In the accompanying drawings, the same reference numerals denote the same or similar elements throughout the views.

### Description of exemplifying embodiments

The following is a description of exemplifying embodiments in accordance with the present invention. It is to be understood that the following description is non-limiting and for the purpose of describing the principles of the invention, and thus, even though particular types of implantable medical devices such as heart stimulators (pacemakers) are described, the invention is also applicable to other types of cardiac rhythm management systems, such as implantable cardioverters (defibrillators), etc.

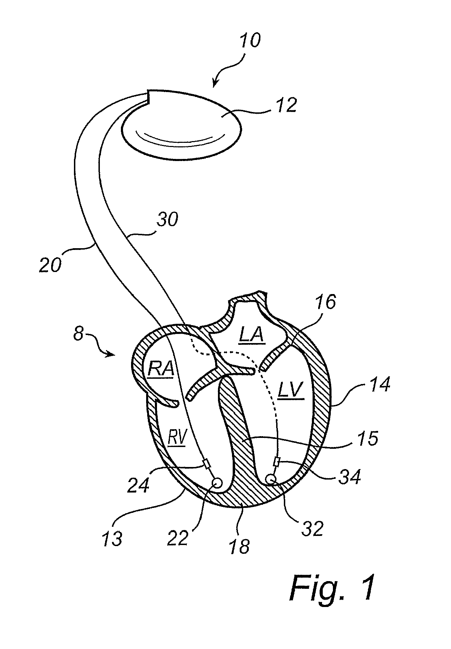

Referring to Fig. 1, there is shown an implantable medical device 10, e.g. a heart-stimulation device according to an embodiment of the present invention. The heart-stimulation device 10 comprises a housing 12 that may be hermetically sealed and biologically inert. Normally, the housing 12 may be conductive, and may as such serve as an electrode. As illustrated in Fig. 1, the heart-stimulation device 10 may electrically communicate with a patient's heart 8 by way of medical leads 20 and 30 suitable for delivering cardiac stimulation, which leads 20 and 30 are connectable to the device 10. The illustrated portions of the patient's heart 8 include the right atrium RA, the right ventricle RV, the left atrium LA, the left ventricle LV, the right ventricular wall 13, the left ventricular wall 14, the ventricle septum 15, the valve plane 16 and the apex 18.

According to the exemplifying embodiment illustrated in Fig. 1, the heart-stimulation device 10 is connected to an implantable right ventricular lead 20, which may be provided with a ventricular tip electrode 22 and a ventricular annular ring electrode 24 in order to provide stimulation therapy to the right ventricle RV and/or sensing cardiac signals from the heart 8. The right ventricular tip electrode 22 is according to the exemplifying illustrated embodiment arranged to be implanted in the endocardium of the right ventricle, for example near the apex 18. Thereby, the tip electrode 22 may become attached to the cardiac wall. The tip electrode 22 may be fixedly mounted in a distal header portion of the lead 20.

Referring further to Fig. 1, the device 10 is further connected to a coronary sinus lead 30 arranged for placement via the coronary sinus in veins located distally from the coronary sinus, so as to place a distal electrode adjacent to the left ventricle LV for providing stimulation therapy to the left ventricle LV. According to the illustrated example, the coronary sinus lead 30 is arranged to receive ventricular cardiac signals from the device 10 and to deliver left ventricular stimulation therapy using a left ventricular tip electrode 32 to the patient's heart 8. The coronary sinus lead 30 may further comprise an annular ring electrode 34 for, e.g., sensing electrical activity related to the left ventricle LV of the heart 8.

Furthermore, for example, a right atrium lead (not shown) may also be included, which may be designed to receive right atrium cardiac signals from the heart-stimulation device 10 and to deliver right atrium stimulation therapy by means of, e.g., a right atrium ring electrode. The present invention covers any number of such medical leads for providing stimulation therapy to and/or sensing cardiac signals at various sites in the heart 8.

The heart-stimulation device 10 may be connected to both bipolar and unipolar medical leads.

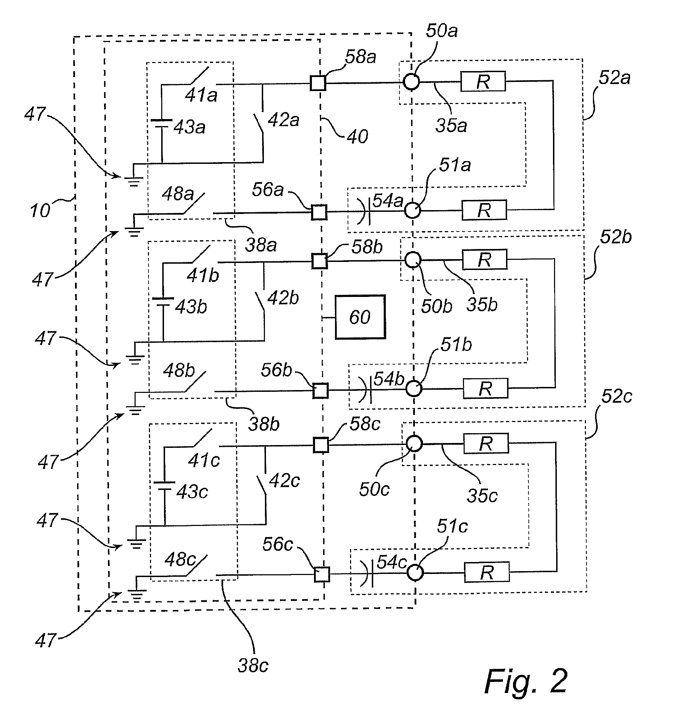

Referring now to Fig. 2, there is shown a schematic circuit diagram of a heart-stimulation device 10 according to an exemplifying embodiment of the present invention. One or more leads, wherein three leads 35a, 35b and 35c are shown in Fig. 2, are electrically connectable to the heart-stimulation device 10 via anodes 50a, 50b, 50c and cathodes 51 a, 51 b, 51 c. The connection of the leads may be effectuated according to a conventional manner. As previously discussed above with reference to Fig. 1, the leads 35a, 35b and 35c may extend into the heart 8 via a vein and comprise one or more electrodes, such as tip electrodes or ring electrodes (not shown in Fig. 2), arranged to, inter alia, transmit stimulation pulses for causing depolarization of cardiac tissue adjacent to the one or more electrodes situated in or about the heart 8. Although Fig. 2 exhibits only three such medical leads 35a, 35b, 35c for transmitting stimulation pulses to in general different sites on or about the heart, the present invention encompasses any number of medical leads, for example four, five, six, eight or ten or more medical leads, each being connectable to the medical device 10, for performing e.g. multi-site cardiac stimulation.

Now, with further reference to Fig. 2, pulse generators 38a, 38b, 38c and dishcarge switching elements 42a, 42b, 42c of a heart-stimulation device 10 will be discussed. According to the depicted embodiment of the present invention, the pulse generators 38a, 38b, 38c and discharge swicthing elements 42a, 42b, 42c are implemented on an integrated circuit (IC) 40. In this example, the IC 40 comprises three anode side switching circuits, each comprising a stimulation switching element 41 a, 41 b and 41 c, respectively, a discharge switching element 42a, 42b and 42c, respectively, and a power source 43a, 43b and 43c, respectively. Each of the switching circuits is connected to a common node 47, typically the ground of the heart-stimulation device 10 (having the voltage 0 V). As illustrated in Fig. 2, the switching circuits may be connected to anodes 50a, 50b and 50c, respectively, via terminals 58a, 58b and 58c, respectively. The power source may for example comprise a battery, such as a lithium iodine battery or a battery based on other lithium systems.

The IC 40 further comprises three cathode side switching elements 48a, 48b and 48c, each being connected to the common node 47. The medical leads 35a, 35b and 35c are connected to load resistance R comprising, inter alia, cardiac tissue and one or more electrodes, such as tip electrodes or ring electrodes (not shown), as previously described with reference to Fig. 1.

Again referring to Fig. 2, the device 10 further comprises three coupling capacitors 54a, 54b, 54c, each of which is capable of being charged and discharged. For example, one or more of the coupling capacitors 54a, 54b, 54c shown in Fig. 2 may comprise, or be, a coupling capacitor in accordance with the illustrated exemplifying embodiment. The coupling capacitors may also comprise several such coupling capacitors, or, optionally or alternatively, one or more of other, similar elements capable of being charged and discharged. As illustrated in Fig. 2, the coupling capacitor 54a may be connected to the cathode 51 a and the IC 40 via the terminal 56a, the coupling capacitor 54b may be connected to the cathode 51 b and the IC 40 via the terminal 56b, and the coupling capacitor 64c may be connected to the cathode 51 c and the IC 40 via the terminal 56c, and thus the coupling capacitors 54a, 54b and 54c may be connected to the cathode side switching elements 48a, 48b and 48c, respectively. One or more of the coupling capacitors may also be arranged integral with the IC (e.g. arranged on the IC).

According to the illustrative example in Fig. 2, the heart-stimulation device 10 may further comprise a control unit 60, which may include, or be, a microprocessor. The control unit 60 may for example be connected to the IC 40, in accordance with the exemplifying device 10 illustrated in Fig. 2, or be implemented integrally on the IC 40. The control unit 60 may be arranged to control, inter alia, the operation of the switching elements 41a-41c, 42a-42c and 48a-48c and other components of the device 10, as well as stimulation-pulse parameters, such as pulse output voltage and pulse duration. The control unit 60 may further include a memory unit (not shown) that in turn may include a random access memory (RAM) and/or a non-volatile memory unit such as a read-only memory (ROM) for data logging, etc. Each element, parameter and/or any other component of the heart-stimulation device 10 may be controlled independently by the control unit 60. The control unit 60 may also be configured to, e.g., sense and/or monitor voltages, currents, etc. of any component of the heart-stimulation device 10 by means of sensing devices (not shown).

Referring now to Fig. 2, the switching elements 41 a and 48a and the power source 43a form a first pulse generator 38a for generating stimulation pulses. By closing the switching elements 41a and 48a, while keeping the switching element 42a open, a current will start flowing from the anode 50a through the load resistance R (inter alia, the patient) to the cathode 51a. At the cathode 51 a the coupling capacitor 54a is arranged in series with the current path, and is charged with the current flowing through the patient during a predetermined stimulation period. In this manner, the anode 50a, the medical lead 35a, and the cathode 51 a form a first stimulation channel 52a for stimulating a stimulation site in or about the patient's heart.

In the same way, the switching elements 41 b and 48b and the power source 43b form a second pulse generator 38b, as by closing the switching elements 41 b and 48b, while keeping the switching element 42b open, a current will start flowing from the anode 50b through the load resistance R (inter alia, through the patient) to the cathode 51 b. At the cathode 51 b the coupling capacitor 54b is charged with the current during a predetermined stimulation period. Analogous to the above, the anode 50b, the medical lead 35b and the cathode 51 b form a second stimulation channel 52b for stimulating a stimulation site in or about the patient's heart.

Furthermore, analogous to the first and second stimulation channels 52a and 52b, the switching elements 41 c and 48c and the power source 43c form a third pulse generator 38c, as by closing the switching elements 41c and 48c, while keeping the switching element 42c open, a current will start flowing from the anode 50c through the load resistance R (inter alia, through the patient) to the cathode 51 c. At the cathode 51 c the coupling capacitor 54c is charged with the current during a predetermined stimulation period. Thus, the anode 50c, the medical lead 35c and the cathode 51 c form a third stimulation channel 52c for stimulating a stimulation site in or about the patient's heart.

Although the embodiments of the present invention described with reference to Fig. 2 only comprise three stimulation channels, the present invention is not limited to only three stimulation channels but rather encompasses embodiments of the present invention comprising any number of stimulation channels, for example two, four, five, six, eight or ten or more stimulation channels for, e.g., delivering cardiac stimulation to multiple sites on or about the patient's heart.

With further reference to Fig. 2, after a stimulation period for one of the stimulation channels, the switching elements 41 a and 48a, the switching elements 41 b and 48c, and/or the switching elements 41 c and 48c, respectively, may be opened, and the charge thus stored at the coupling capacitor 54a, 54b and 54c, respectively, may subsequently be discharged through the load resistance R (inter alia, through the patient) during a predetermined discharge period. Discharge of the coupling capacitors may be performed by connecting the anode side and the cathode side of the coupling capacitors 54a, 54b, 54c to a common electrical node (for example, the device ground 47) during a predetermined discharge period, i.e. by closing the switching elements 42a and 48a, 42b and 48b and/or 42c and 48c, respectively, while keeping the switching elements 41 a, 41 b and/or 41 c, respectively, in an open state during a predetermined discharge period. In order to maintain charge neutrality in the heart-stimulation device 10, equal amounts of charge must be flowing in both directions in each stimulation channel.

For implementing the various switching elements on an IC, transistors such as PMOS transistors and NMOS transistors may be utilized. The components and/or circuits of a heart-stimulation device according to the present invention may accordingly be implemented on a p-doped semiconductor substrate, such as an IC according to the examples described in the foregoing and in the following, which IC is connected to a suitable node, e.g., the electrical ground of the device. On the semiconductor substrate, n-doped wells may be formed for accommodating the transistor structures. With this topology, a parasitic diode structure may be formed between the n-well and the p-substrate whenever the n-well potential attains a value below a diode threshold voltage relative to the device ground. By "parasitic structure", it is meant a portion of a semiconductor device structurally resembling some other semiconductor device and which may cause the semiconductor device to enter unintended modes of operation when subjected to conditions outside its normal operating range. Semiconductor devices in general exhibit such parasitic structures. In other words, in the context of the present application, by the term "parasitic" it is referred to an unwanted circuit element that is an unavoidable adjunct of a wanted circuit element. For example, whenever an n-doped region and a p-doped region on an IC are situated in close proximity, a parasitic p-n diode may be formed in the p-n junction. If a large enough bias is placed on the junction, a parasitic p-n diode is going to be formed, and current will flow over the parasitic p-n diode. During normal operation of the device, such a formation of a parasitic p-n diode may be undesirable.

This situation is illustrated in Fig. 3, which is a section of the circuit as shown in Fig. 2, which section comprises the switching element 48 and some of its neighboring components. The same reference numerals in Figs. 2 and 3 denote identical or similar components. More specifically, Fig. 3 is a schematic circuit diagram wherein the switching element 48 has been implemented as a component comprising an NMOS transistor having a source S, a drain D, a base B and a gate G, wherein the source S is connected to the semiconductor substrate of the IC and the drain D is connected to the coupling capacitor 54a. Thus, the NMOS transistor in Fig. 3 is employed as a switch. A parasitic p-n diode, which thus may be formed, is indicated at the reference numeral 62a.

Consider now the scenario in which cardiac stimulations are required substantially simultaneously at multiple sites in or about a heart, and where multiple stimulation channels, each comprising a coupling capacitor, are used, and the stimulation periods are required to be shorter than the respective discharge periods. This scenario is applicable e.g. to cardiac resynchronization therapy sessions. In this case, the potentials on the coupling capacitors may become high. When using the device ground as the common discharge node and using multiple coupling capacitors, which are not fully discharged one by one, the n-well potential may become less than a parasitic diode threshold voltage below the ground potential, which in turn leads to current leaking from the substrate to the n-well. For example, when performing cardiac stimulation in a two-channel system with short timing intervals between the pulses delivered to the left and right ventricle of a heart, there may not be enough time between the stimulation pulses to perform a complete discharge of the first coupling capacitor of the first stimulation channel before the next stimulation pulse is delivered. Thus, there will be a non-zero voltage V₁ with respect to system ground V_{SS} over the first coupling capacitor of the first stimulation channel when the next stimulation pulse begins. This will have the effect that when the discharge period of the first stimulation channel begins, after the second stimulation pulse has been delivered, the voltage of the anodic side of the first coupling capacitor will become substantially equal to system ground V_{SS} and voltage of the cathode side of the first coupling capacitor will be brought down to value below system ground, namely to V_{SS}-V₁. For the cases when there is a high voltage V₁ (with respect to V_{SS}) left on the first coupling capacitor when the next stimulation pulse begins, the magnitude of the voltage of the cathode side of the first coupling capacitor V_{SS}-V₁ may exceed the threshold voltage of the parasitic p-n diode related to the IC's semiconductor substrate, at which point charge will leak from the first coupling capacitor to the semiconductor substrate through the parasitic p-n diode. In order to maintain charge neutrality, the charge thus lost from the first coupling capacitor should be discharged back through the stimulation channel (through the lead) during the discharge sequence, and thus, according to the above scenario, charge neutrality of the stimulation system may not be maintained after a stimulation sequence has been performed.

The above-described scenario also applies to a stimulation system employing more than two stimulation channels.

As already discussed in the foregoing, in the context of the present invention it is meant by "stimulation sequence" a sequence of events comprising stimulation during a stimulation period, including delivery of stimulation pulses via stimulation channels, and discharge during a discharge period, including discharge of the stimulation channels. This is illustrated in Fig. 4, which are schematic voltage diagrams of a stimulation sequence S1 in a three-channel stimulation system according to an exemplifying embodiment of the present invention, where each voltage diagram from the top to bottom of Fig. 4 corresponds to a stimulation channel. The vertical axis of each of the voltage diagrams corresponds to the voltage (in arbitrary units) over a load resistance (comprising, inter alia, the heart of a patient). The horizontal axis of each of the voltage diagrams corresponds to time (in arbitrary units). As schematically illustrated in Fig. 4, the stimulation sequence S1 comprises delivery S2 of stimulation pulses and discharge S3 of the stimulation channels.

Referring now again to Fig. 3 and considering the above-described scenario, the magnitude of the voltage on the drain D of the NMOS transistor used for implementing the switching element 48a may exceed the threshold voltage of the parasitic p-n diode 62a related to the semiconductor substrate of the IC (i.e. ground 47) when the discharge period of the first stimulation channel begins, at which charge may leak from the first coupling capacitor 54a to the semiconductor substrate through the parasitic p-n diode 62a.

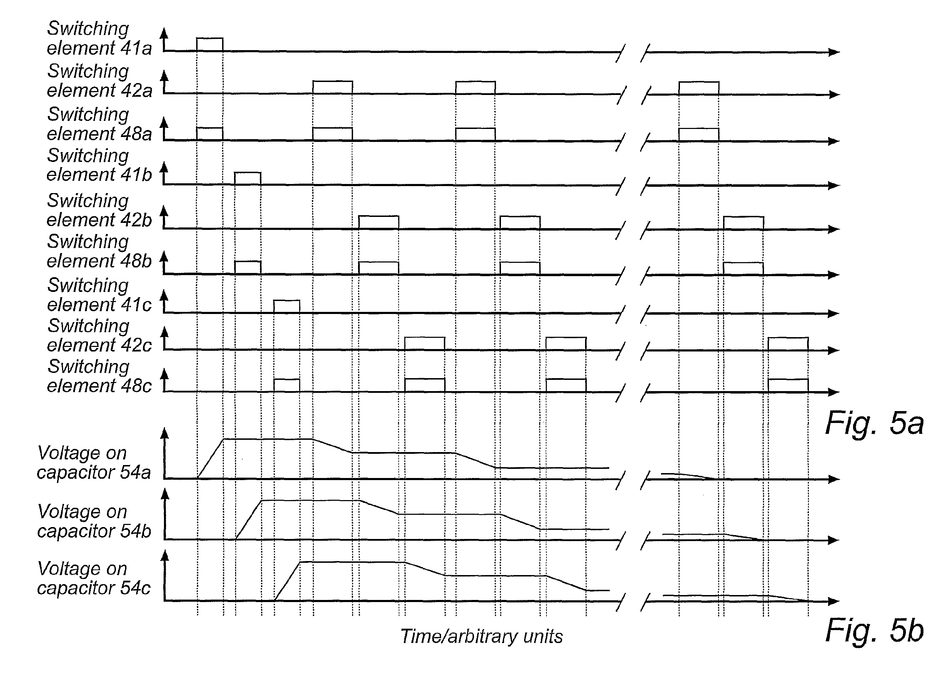

As have been discussed above and in the following, the present invention addresses this problem by providing an improved heart-stimulation device that reduces or eliminates the risk of charge neutrality in the heart-stimulation device not being maintained at the end of a stimulation sequence due to charge leaking from a coupling capacitor to a semiconductor substrate on which the components and/or circuits of the heart-stimulation device are implemented. The general principles of the present invention will now be described with reference to an exemplifying embodiment of the present invention depicted in Figs. 5a and 5b.

Fig. 5a is a schematic timing diagram showing exemplifying stimulation sequences according to an exemplary embodiment of the present invention. With reference to Fig. 2 as discussed above, a non-zero value on the vertical axes in Fig. 5a indicates that the respective switching element, as indicated by the captions of the vertical axes, is in an open state, and a vanishing, substantially zero value on the vertical axes in Fig. 5a indicates that the respective switching element is in a closed state. The horizontal axes in Fig. 5a indicate time (in arbitrary units).

Referring to Fig. 2, the control of the switching elements, for example according to the timing diagram in Fig. 5a, may be performed by means of a control unit 60.

With further reference to Fig. 2, Fig. 5b is a schematic voltage diagram showing the voltage (in arbitrary units) of the coupling capacitors 54a, 54b and 54c, respectively, as a function of time (in arbitrary units). The horizontal axes in Fig. 5b correspond to the horizontal axes in Fig. 5a.

Referring now again to Fig. 5a, the stimulation pulses on the respective channels are delivered sequentially by, during a predetermined stimulation period, concurrently keeping the switching elements 41 a and 48a in an open state while keeping the switching element 42a in a closed state. Then, the switching elements 41a and 48a are closed, and, during a predetermined stimulation period, the switching elements 41 b and 48b are concurrently kept in an open state while the switching element 42b is kept in a closed state. Finally, the switching elements 41 b and 48b are closed, and, during a predetermined stimulation period, the switching elements 41 c and 48c are concurrently kept in an open state while the switching element 42c is kept in a closed state. In this way, the stimulation pulses in the respective stimulation channels are delivered sequentially and temporally non-overlapping.

Referring to Fig. 5b, the voltages on the coupling capacitors 54a, 54b and 54c, respectively, are accordingly increased as the coupling capacitors 54a, 54b and 54c are charged during the delivery of the stimulation pulses.

Again referring to Fig. 5a, after the stimulation pulses for each of the stimulation channels have been delivered, the voltage on the coupling capacitors 54a, 54b and 54c are step-wise decreased by repeatedly partially discharging the coupling capacitors 54a, 54b and 54c according to a predetermined, temporally non-overlapping discharge sequence, an exemplifying sequence of which is illustrated in Fig. 5b, such that each partial discharge has a predetermined discharge period. The process of repeatedly partially discharging the coupling capacitors 54a, 54b and 54c is performed until every coupling capacitor 54a, 54b, 54c has a voltage that is less than a predetermined voltage level. For example, the process of repeatedly partially discharging the coupling capacitors 54a, 54b and 54c is performed until every coupling capacitor 54a, 54b, 54c has a voltage that is substantially zero, or alternatively less than a threshold voltage of a parasitic structure, such as a parasitic p-n diode, formed between the semiconductor substrate of the IC 40 and the respective coupling capacitor 54a, 54b, 54c.

Although the process of partially discharging the coupling capacitors 54a, 54b and 54c of the stimulation channels 52a, 52b and 52c, respectively, is carried out in a periodically sequential manner according to the embodiment described with reference to the timing and voltage diagrams depicted in Figs. 5a and 5b, the process of partially discharging the stimulation channels may be carried out according to any temporally non-overlapping sequence, starting with a first stimulation channel, conforming with the general principles of the present invention. For example, the control unit 60 may be adapted to repeatedly partially discharge the coupling capacitors of the stimulation channels one stimulation at a time while alternating between the stimulation channels.

The control unit 60 may be adapted such that the discharge period of each partial discharge of the coupling capacitors 54a, 54b, and 54c is such that by a partial discharge the voltage of the respective coupling capacitor decreases by a voltage that is less than a threshold voltage of a parasitic structure, for example a parasitic p-n diode, formed between the semiconductor substrate of the IC and the respective coupling capacitor.

In this manner, by the step-wise partial discharging of the coupling capacitors 54a, 54b, 54c that is performed repeatedly, it is prevented or inhibited that the cathode side of any of the coupling capacitors 54a, 54b, 54c attains a voltage below system ground and below a threshold voltage of a parasitic structure, such as a parasitic p-n diode, formed between the semiconductor substrate of the IC 40 and the respective coupling capacitor 54a, 54b, 54c. Thus, the charge loss from the coupling capacitors 54a, 54b, 54c can be reduced or eliminated, and charge neutrality in the heart-stimulation device, or more specifically in the respective stimulation channels, may be maintained after each stimulation sequence has been performed.

Furthermore, the control unit 60 may be adapted to perform a comparison of the change in the voltage of one or more of the coupling capacitors 54a, 54b and 54c resulting from a partial discharge of the stimulation channel to which the respective coupling capacitor is associated, in accordance with the above discussion with reference to Figs. 5a and 5b. Subsequently, the control unit 60 may be further adapted to adjust the discharge period that is employed for the respective stimulation channel on the basis of the thus performed comparison. Accordingly, the magnitude of the adjustment of a partial discharge period associated with a particular stimulation channel thus depends on the so determined change in voltage of the coupling capacitor of the particular stimulation channel, resulting from a partial discharge of the coupling capacitor associated therewith. The adjustment of the partial discharge period for a particular stimulation channel may be performed at any suitable time, for example within a time period during which a sequence of partial discharges of the coupling capacitor associated with the stimulation channel is being carried out. In other words, the length of the partial discharge period may be adjusted dynamically during operation of the heart-stimulation device. The adjustment of the length of the discharge period may typically range from about 0.2 ms to about 1 ms, depending on the initial length of the discharge period.

As the process of repeatedly partially discharging a particular stimulation channel, as discussed above with reference to Figs. 5a and 5b, is performed until the voltage of the coupling capacitor associated therewith is below a predetermined voltage level, the number of partial discharges carried out during the discharge sequence for the particular stimulation channel depends on the length of the partial discharge period. It is generally desirable to set the length of the partial discharge period for each stimulation channel such that the temporal variation between the voltages on the different coupling capacitors during the discharge sequence is small or minimal (cf. Figs. 5a and 5b). This is in general achieved by setting the length of the partial discharge period for each of the stimulation channels to a low value, typically to a value ranging from about 0.5 ms to about 1 ms, or in some cases to a value ranging from about 0.2 ms or lower to about 0.5 ms. Alternatively or optionally, in case a large temporal variation between the voltages on the different coupling capacitors during the discharge sequence is detected (e.g. by the control unit), the length of the discharge period for one or more of the coupling capacitors may be adjusted such that the temporal variation between the voltages on the different coupling capacitors during the discharge sequence becomes smaller.

In some situations it may be appropriate to control the partial discharges for the stimulation channels such that the length of the total discharge period for each of the stimulation channels corresponds to a predetermined value, for example to a value of about 20 ms or less.

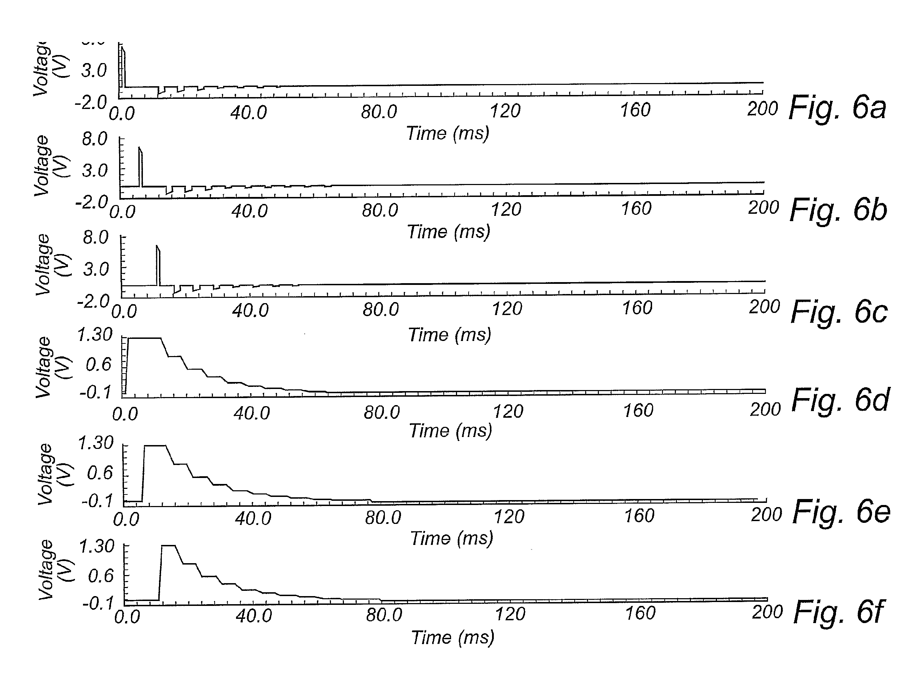

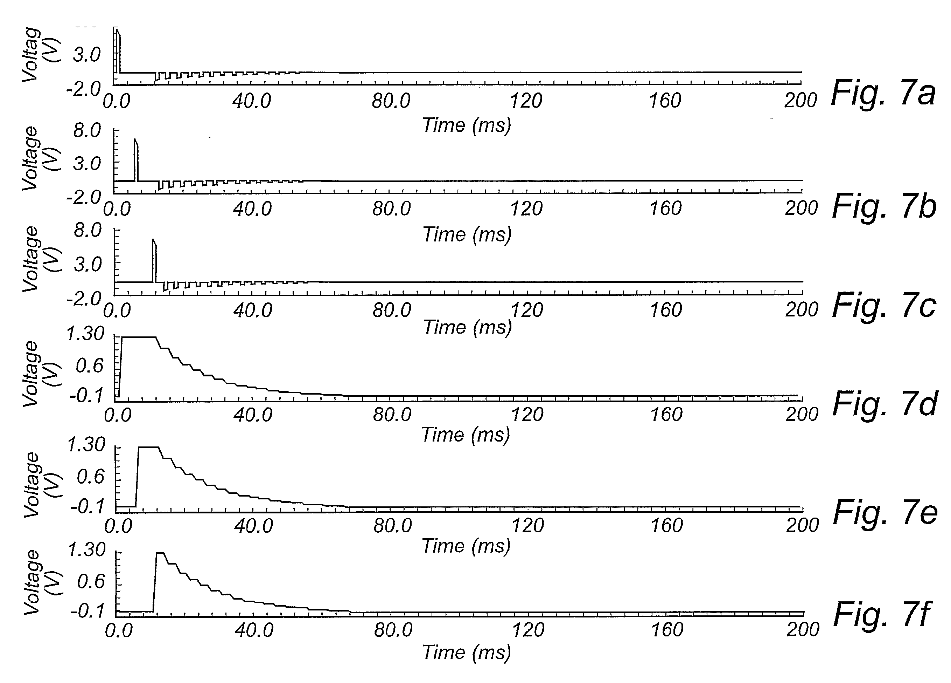

Figs. 6a-6f and 7a-7f are voltage diagrams illustrating two exemplifying embodiments of the present invention, respectively, wherein two different discharge periods for repeatedly partially discharging coupling capacitors of the respective stimulation channels are utilized.

First referring to Figs. 6a-6c, there is shown the results of a computer simulation of a stimulation sequence utilizing three different stimulation channels in a heart-stimulation device according to an exemplifying embodiment of the present invention. The three different stimulation channels may be used for e.g. delivering stimulation therapy to in general different sites located on or about the heart of a patient. In each of Figs. 6a-6c there is shown the voltage over a load resistance R (comprising, inter alia, the patient) as a function of time for a stimulation in the respective stimulation channel. As depicted in Figs. 6a-6c, the three stimulation pulses are delivered sequentially.

After the stimulation pulses have been delivered, short periods of partial discharge of the stimulation channels begin in accordance with an embodiment of the present invention. This is depicted in Figs. 6d-6f, which show the voltages over the coupling capacitors for the three stimulation channels, respectively. In Figs. 6a-6f, Fig. 6a and Fig. 6d correspond to a first stimulation channel, Fig. 6b and Fig. 6e correspond to a second stimulation channel, and Fig. 6c and Fig. 6f correspond to a third stimulation channel. According to the illustrated embodiment in Figs. 6d-6f, a discharge period of about 2 ms is used for each of the stimulation channels throughout the discharge sequence. Thus, in the example depicted in Figs. 6d-6f, the discharge period of each stimulation channel is fixed throughout the discharge sequence and is the same as the discharge period of the other two stimulation channels.

Second, referring to Figs. 7a-7f, there is shown voltage diagrams illustrating an exemplifying embodiment of the present invention, analogous to the voltage diagrams in Figs. 6a-6f, respectively. In Figs. 7a-7f, Fig. 7a and Fig. 7d correspond to a first stimulation channel, Fig. 7b and Fig. 7e correspond to a second stimulation channel, and Fig. 7c and Fig. 7f correspond to a third stimulation channel. The illustrated embodiment in Figs. 7d-7f differs from the embodiment described with reference to Figs. 6a-6f in that a discharge period of about 1 ms is used for each of the stimulation channels throughout the discharge sequence. Thus, in the example depicted in Figs. 7d-7f, the discharge period of each stimulation channel is fixed throughout the discharge sequence and is the same as the discharge period of the other two stimulation channels.

Typically, the coupling capacitors of the heart-stimulation device according to the present invention may be implemented as capacitors. For a charge Q, the capacitance C is inversely proportional to the voltage V; C=Q/V. Therefore, on one hand the charge leakage as described above with reference to the two-channel stimulation system scenario will increase if the capacitance of the coupling capacitors is decreased. On the other hand, if the capacitance of the coupling capacitors is increased too much, the coupling capacitors are going to take a (too) long time to charge. In embodiments of the present invention, the capacitance of the coupling capacitors may be set to values ranging from about 4 µF to about 50 µF.

The control of the discharge sequences of the stimulation channels (e.g. the control of the switching elements) according to the different exemplifying embodiments of the present invention as have been described in the foregoing may be implemented in a hardware state machine supporting firmware controlling the switching elements. Alternatively, the control of the switching elements may be fully implemented in firmware.

Although exemplary embodiments of the present invention has been described herein, it should be apparent to those having ordinary skill in the art that a number of changes, modifications or alterations to the invention as described herein may be made. Thus, the above description of the invention and the accompanying drawings are to be regarded as non-limiting examples of the invention and the scope of protection is defined by the appended claims.

## Claims

1. An implantable heart-stimulation device (10), comprising:
a pulse generator (38a, 38b, 38c) arranged to generate stimulation pulses, said pulse generator being connectable to at least one medical lead (20, 30; 35a, 35b, 35c) including a plurality of electrodes;
a control unit (60), and
a plurality of stimulation channels (52a, 52b, 52c) for delivering said stimulation pulses via said electrodes, each stimulation channel including at least one coupling capacitor (54a, 54b, 54c);
wherein the control unit (60) is adapted to:
perform a partial discharge of coupling capacitors (54a, 54b, 54c) of said stimulation channels (52a, 52b, 52c),
wherein each partial discharge has a predetermined discharge period, **characterized in that** the control unit (60) is adapted to repeatedly perform the partial discharge according to a predetermined temporally non-overlapping sequence until each of said coupling capacitors has a voltage less than a predetermined voltage level.

2. The device according to claim 1, wherein the control unit (60) is further adapted to repeatedly partially discharge coupling capacitors (54a, 54b, 54c) of said stimulation channels (52a, 52b, 52c) one stimulation channel at a time while alternating between said stimulation channels.

3. The device according to claim 1 or 2, wherein the control unit (60) is further adapted to control said partial discharges for said stimulation channels (52a, 52b, 52c) such that the total discharge period for each stimulation channel corresponds to a predetermined total discharge period.

4. The device according to any one of the preceding claims, wherein each of the plurality of stimulation channels (52a, 52b, 52c) includes an anode (50a, 50b, 50c) and a cathode (51 a, 51b, 51 c), and a coupling capacitor (54a, 54b, 54c) is located between the anode and the cathode, and wherein the partial discharge of a coupling capacitor of the respective stimulation channel is effectuated by connecting the anode side and the cathode side of said coupling capacitor to a common electrical node (47) during a predetermined discharge period.

5. The device according to claim 4, wherein the control unit (60) is further adapted to:
comparing the potential of the anode side and the cathode side of a coupling capacitor (54a, 54b, 54c) of at least one stimulation channel (52a, 52b, 52c); and
on the basis of said comparison, adjusting the discharge period for said at least one stimulation channel.

6. The device according to any one of the preceding claims, wherein the control unit (60) is further adapted to control said pulse generator (38a, 38b, 38c) to deliver stimulation pulses via said stimulation channels (52a, 52b, 52c) according to a predetermined sequence.

7. The device according to any one of the preceding claims, wherein components and/or circuits of said device are implemented on a semiconductor substrate (40), and wherein the discharge period is such that by the partial discharge the voltage of the at least one coupling capacitor (54a, 54b, 54c) of the respective stimulation channel (52a, 52b, 52c) decreases by a voltage less than a threshold voltage of a parasitic structure (62a) formed between the semiconductor substrate and said coupling capacitor.

8. The device according to any one of the preceding claims, wherein the control unit (60) is further adapted to:
comparing the change in voltage of a coupling capacitor (54a, 54b, 54c) of at least one of said stimulation channels (52a, 52b, 52c) resulting from a partial discharge of said coupling capacitor; and
on the basis of said comparison, adjusting the discharge period for said at least one stimulation channel.

9. The device according to any one of the preceding claims, wherein the control unit (60) is further adapted to:
comparing voltages of coupling capacitors (54a, 54b, 54c) of said stimulation channels (52a, 52b, 52c); and
on the basis of said comparison, adjusting the discharge period for one or more of said stimulation channels.

## Patentansprüche

1. Implantierbare Herzstimulationsvorrichtung (10), umfassend:
einen Impulsgenerator (38a, 38b, 38c), der so angeordnet ist, dass er Stimulationsimpulse erzeugt, wobei der Impulsgenerator mit mindestens einer medizinischen Leitung (20, 30; 35a, 35b, 35c) verbindbar ist, die eine Vielzahl von Elektroden aufweist;
eine Steuereinheit (60), und
eine Vielzahl von Stimulationskanälen (52a, 52b, 52c) zum Abgeben der Stimulationsimpulse über die Elektroden, wobei jeder Stimulationskanal mindestens einen Koppelkondensator (54a, 54b, 54c) aufweist;
wobei die Steuereinheit (60) so ausgelegt ist, dass sie:
eine Teilentladung von Koppelkondensatoren (54a, 54b, 54c) der Stimulationskanäle (52a, 52b, 52c) durchführt,
wobei jede Teilentladung eine vorher festgelegte Entladezeit aufweist, **dadurch gekennzeichnet, dass** die Steuereinheit (60) so ausgelegt ist, dass sie die Teilentladung entsprechend einer vorher festgelegten, sich zeitlich nicht überlappenden Abfolge wiederholt durchführt, bis jeder der Koppelkondensatoren eine Spannung von weniger als einem vorher festgelegten Spannungspegel aufweist.

2. Vorrichtung nach Anspruch 1, wobei die Steuereinheit (60) ferner so ausgelegt ist, dass sie Koppelkondensatoren (54a, 54b, 54c) der Stimulationskanäle (52a, 52b, 52c) einen Stimulationskanal nach dem anderen wiederholt teilweise entlädt, während sie zwischen den Stimulationskanälen wechselt.

3. Vorrichtung nach Anspruch 1 oder 2, wobei die Steuereinheit (60) ferner so ausgelegt ist, dass sie die Teilentladungen für die Stimulationskanäle (52a, 52b, 52c) so steuert, dass die Gesamtentladungszeit für jeden Stimulationskanal einer vorher festgelegten Gesamtentladungszeit entspricht.

4. Vorrichtung nach einem der vorhergehenden Ansprüche, wobei jeder von der Vielzahl von Stimulationskanälen (52a, 52b, 52c) eine Anode (50a, 50b, 50c) und eine Kathode (51 a, 51 b, 51 c) aufweist und ein Koppelkondensator (54a, 54b, 54c) zwischen der Anode und der Kathode angeordnet ist, und wobei die Teilentladung eines Koppelkondensators des jeweiligen Stimulationskanals durch Verbinden der Anodenseite und der Kathodenseite des Koppelkondensators mit einem gemeinsamen elektrischen Knotenpunkt (47) während einer vorher festgelegten Entladungszeit bewirkt wird.

5. Vorrichtung nach Anspruch 4, wobei die Steuereinheit (60) ferner so ausgelegt ist, dass sie:
das Potenzial der Anodenseite und der Kathodenseite eines Koppelkondensators (54a, 54b, 54c) von mindestens einem Stimulationskanal (52a, 52b, 52c) vergleicht; und
auf der Grundlage des Vergleichs die Entladezeit für den mindestens einen Stimulationskanal anpasst.

6. Vorrichtung nach einem der vorhergehenden Ansprüche, wobei die Steuereinheit (60) ferner so ausgelegt ist, dass sie den Impulsgenerator (38a, 38b, 38c) so steuert, dass er Stimulationsimpulse über die Stimulationskanäle (52a, 52b, 52c) entsprechend einer vorher festgelegten Abfolge abgibt.

7. Vorrichtung nach einem der vorhergehenden Ansprüche, wobei Bauteile und/oder Schaltungen der Vorrichtung auf einem Halbleiterträgermaterial (40) ausgeführt sind, und wobei die Entladezeit derart ist, dass durch die Teilentladung die Spannung des mindestens einen Koppelkondensators (54a, 54b, 54c) des jeweiligen Stimulationskanals (52a, 52b, 52c) um eine Spannung sinkt, die niedriger ist als eine Schwellenspannung einer parasitären Struktur (62a), die zwischen dem Halbleiterträgermaterial und dem Koppelkondensator ausgebildet ist.

8. Vorrichtung nach einem der vorhergehenden Ansprüche, wobei die Steuereinheit (60) ferner so ausgelegt ist, dass sie:
die Spannungsänderung eines Koppelkondensators (54a, 54b, 54c) von mindestens einem der Stimulationskanäle (52a, 52b, 52c) vergleicht, die von einer Teilentladung des Koppelkondensators herrührt; und
auf der Grundlage des Vergleichs die Entladezeit für den mindestens einen Stimulationskanal anpasst.

9. Vorrichtung nach einem der vorhergehenden Ansprüche, wobei die Steuereinheit (60) ferner so ausgelegt ist, dass sie:
Spannungen von Koppelkondensatoren (54a, 54b, 54c) der Stimulationskanäle (52a, 52b, 52c) vergleicht; und
auf der Grundlage des Vergleichs die Entladezeit für einen oder mehrere der Stimulationskanäle anpasst.

## Revendications

1. Dispositif de stimulation cardiaque implantable (10), comprenant :
un générateur d'impulsions (38a, 38b, 38c) agencé pour générer des impulsions de stimulation, ledit générateur d'impulsions pouvant être raccordé à au moins une dérivation médicale (20, 30 ; 35a, 35b, 35c) comportant une pluralité d'électrodes ;
une unité de commande (60), et
une pluralité de canaux de stimulation (52a, 52b, 52c) destinés à délivrer lesdites impulsions de stimulation par le biais desdites électrodes, chaque canal de stimulation comportant au moins un condensateur de couplage (54a, 54b, 54c) ;
ladite unité de commande (60) étant adaptée pour :
effectuer une décharge partielle de condensateurs de couplage (54a, 54b, 54c) desdits canaux de stimulation (52a, 52b, 52c),
chaque décharge partielle présentant une durée de décharge prédéterminée, **caractérisé en ce que** l'unité de commande (60) est adaptée pour effectuer de façon répétée la décharge partielle selon une séquence prédéterminée ne se chevauchant pas dans le temps jusqu'à ce que chacun desdits condensateurs de couplage ait une tension inférieure à un niveau de tension prédéterminé.

2. Dispositif selon la revendication 1, dans lequel l'unité de commande (60) est en outre adaptée pour décharger partiellement de façon répétée des condensateurs de couplage (54a, 54b, 54c) desdits canaux de stimulation (52a, 52b, 52c) un canal de stimulation à la fois tout en alternant entre lesdits canaux de stimulation.

3. Dispositif selon la revendication 1 ou 2, dans lequel l'unité de commande (60) est en outre adaptée pour commander lesdites décharges partielles pour lesdits canaux de stimulation (52a, 52b, 52c) de telle manière que la durée de décharge totale pour chaque canal de stimulation correspond à une durée de décharge totale prédéterminée.

4. Dispositif selon l'une quelconque des revendications précédentes, dans lequel chacun de la pluralité de canaux de stimulation (52a, 52b, 52c) comporte une anode (50a, 50b, 50c) et une cathode (51 a, 51 b, 51 c), et un condensateur de couplage (54a, 54b, 54c) est situé entre l'anode et la cathode, et dans lequel la décharge partielle d'un condensateur de couplage du canal de stimulation respectif est accomplie en raccordant le côté anode et le côté cathode dudit condensateur de couplage à un noeud électrique (47) commun pendant une durée de décharge prédéterminée.

5. Dispositif selon la revendication 4, dans lequel l'unité de commande (60) est en outre adaptée pour :
comparer le potentiel du côté anode et du côté cathode d'un condensateur de couplage (54a, 54b, 54c) d'au moins un canal de stimulation (52a, 52b, 52c) ; et
sur la base de ladite comparaison, ajuster la durée de décharge pour ledit au moins un canal de stimulation.

6. Dispositif selon l'une quelconque des revendications précédentes, dans lequel l'unité de commande (60) est en outre adaptée pour commander ledit générateur d'impulsions (38a, 38b, 38c) pour qu'il délivre des impulsions de stimulation par le biais desdits canaux de stimulation (52a, 52b, 52c) selon une séquence prédéterminée.

7. Dispositif selon l'une quelconque des revendications précédentes, dans lequel des composants et/ou circuits dudit dispositif sont mis en oeuvre sur un substrat de semi-conducteur (40), et dans lequel la durée de décharge est telle que, en raison de la décharge partielle, la tension de l'au moins un condensateur de couplage (54a, 54b, 54c) du canal de stimulation (52a, 52b, 52c) respectif diminue d'une tension inférieure à une tension seuil d'une structure parasitaire (62a) formée entre le substrat de semi-conducteur et ledit condensateur de couplage.

8. Dispositif selon l'une quelconque des revendications précédentes, dans lequel l'unité de commande (60) est en outre adaptée pour :
comparer le changement de tension d'un condensateur de couplage (54a, 54b, 54c) d'au moins l'un desdits canaux de stimulation (52a, 52b, 52c) qui résulte d'une décharge partielle dudit condensateur de couplage ; et
sur la base de ladite comparaison, ajuster la durée de décharge pour ledit au moins un canal de stimulation.

9. Dispositif selon l'une quelconque des revendications précédentes, dans lequel l'unité de commande (60) est en outre adaptée pour :
comparer des tensions de condensateurs de couplage (54a, 54b, 54c) desdits canaux de stimulation (52a, 52b, 52c) ; et
sur la base de ladite comparaison, ajuster la durée de décharge pour un ou plusieurs desdits canaux de stimulation.
